# EUROPEAN PATENT SPECIFICATION

(11) **EP 1 624 933 B1**
(45) Date of publication and mention of the grant of the patent: **18.07.2007**
(21) Application number: 04732257.3
(22) Date of filing: 12.05.2004
(51) Int. Cl.: A61N 5/10

(54) **METHOD AND SYSTEM FOR AUTOMATIC BEAM ALLOCATION IN A MULTI-ROOM PARTICLE BEAM TREATMENT FACILITY**
VERFAHREN UND SYSTEM ZUR AUTOMATISCHEN STRAHLZUWEISUNG IN EINER TEILCHENSTRAHLENTHERAPIEANLAGE MIT MEHREREN RÄUMEN
PROCEDE ET SYSTEME D'ALLOCATION AUTOMATIQUE D'UN FAISCEAU DANS UNE INSTALLATION DE TRAITEMENT DE FAISCEAUX DE PARTICULES A PIECES MULTIPLES

(30) Priority: 13.05.2003 US 470382 P
(43) Date of publication of application: 15.02.2006
(73) Proprietor: ION BEAM APPLICATIONS S.A., 1348 Louvain-la-Neuve (BE)
(72) Inventor: LEYMAN, Didier, B-1367 RAMILIES (BE); THIRIONET, Philippe, B-1348 LOUVAIN-LA-NEUVE (BE); DECROCK, Patrick, B-3010 KESSEL-LO (BE); FLORQUIN, Renaud, B-1480 TUBIZE (BE)
(74) Representative: Van Malderen, Joëlle
(86) International application number: PCT/BE2004/000070
(87) International publication number: WO 2004/101070

(56) References cited:
- US-A- 5 260 581

## Description

### Field of the invention

The present invention is related to a method and accompanying software, as well as a system for scheduling a beam in multi-room particle beam treatment facilities.

### State of the art

Particle beam treatment, in particular proton beam treatment, is widely used in medical environments. Facilities offering such treatments often consist of one beam source, such as a cyclotron, and a plurality of treatment rooms, each equipped for example with a rotary gantry or a fixed beam apparatus for irradiating a patient who is positioned inside the treatment room. Beam transport lines connect the cyclotron with the treatment rooms. Such beam lines consist of a succession of magnets for conditioning and deflecting the beam and guiding it towards one of the treatment rooms.

In existing facilities, the allocation of the beam to a treatment room is performed manually. With allocation is meant the selecting of a particular beam line through which an irradiation session is to take place. The beam is allocated during the whole of the treatment cycle, but the beam is not actually irradiating during this whole time.

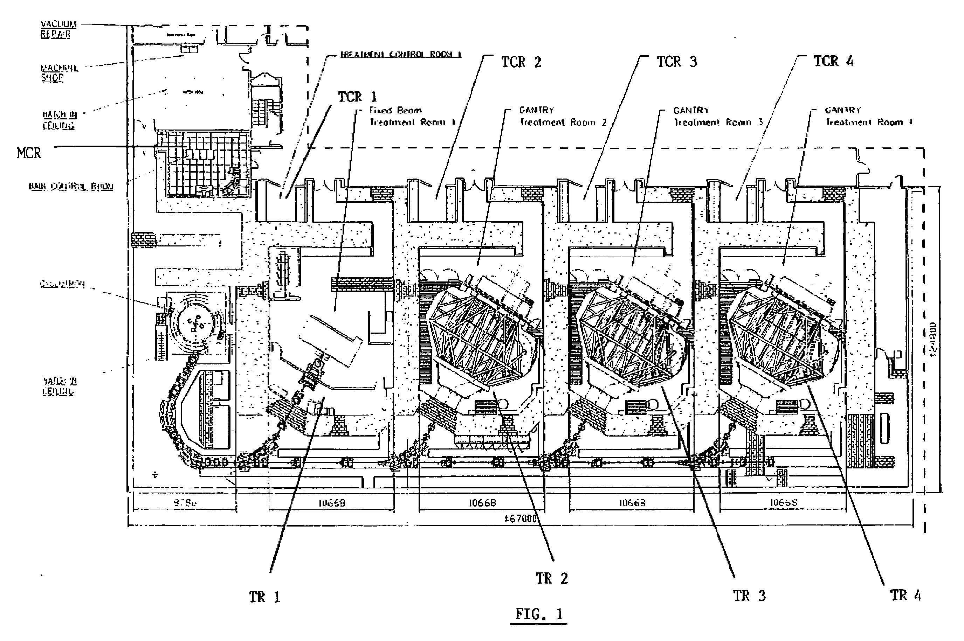

A typical facility is shown in the enclosed figure 1, and comprises four treatment rooms, TR1 to TR4. Treatment control rooms TCR1 to TCR4 are present near every treatment room. A TCR computer is located in every TCR. The TCRs comprise the operator interface and communication equipment and screens with which the TCR-operator may perform the treatment cycle, in communication with the main control room MCR, wherein one operator supervises and controls the treatments taking place in the various TRs. A MCR computer is located in the MCR and is linked with the TCR computer through a high speed local area network.

Any treatment starts with a beam request coming from one of the TR/TCRs In the known methods wherein the allocation is done manually, such a request is acknowledged by the operator in the MCR, which will then manually allocate the beam to the relevant treatment room, when the beam becomes available.

US-A-5260581 discloses a method of treatment room selection verification in a radiation beam therapy system comprising a plurality of treatment rooms. This document addresses the problem of security by verifying the authenticity of a beam request signal. However, this document does not discuss the problem of allocating the beam when multiple beam requests are issued with various characteristics and requirements

The known manual mode holds a risk of human error. Also, an optimum use of the various treatment rooms is not guaranteed, nor is it certain that the MCR operator will allocate a given beam line with the priority requested. Especially when many requests follow each other in close succession, the risk of error increases. Communication among many operators and therapists, separated from each other through thick shielding walls, may lead to frustration, inefficiency and errors. Also, unnecessary time delays will occur.

### Summary of the invention

The invention is related to a method and system as described in the appended claims, and to a software tool for performing this method.

According to the method of the invention, the MCR-operator does not intervene in the allocation of the relevant beam line. Instead, when the beam is available, the allocation takes place automatically. If the beam is not available, the request is automatically put on a waiting list (queued). The position of the request on the list, i.e. the order in which the requests are listed, depends on a priority level connected to the request. For a high priority request, a forced release of the beam can be performed, depending on the priority level of the request for which the beam had been allocated. When the beam operation is finished, the method of the invention allows automatic beam release, depending on the priority with which the beam had been requested.

The invention is equally related to software which governs this automatic beam scheduling method, and allows switching to the known manual mode.

### Brief description of the drawings

Fig. 1 describes an irradiation facility to which the method of the invention can be applied.

Fig. 2 shows the flow diagram according to which the method of the invention takes place.

Fig. 3 to 5 illustrate the difference between allocation and use of the beam for a service priority request, a high priority request, and a normal priority request, respectively. Fig. 5 illustrates the automatic beam release in case of a normal priority request.

Fig. 6 to 11 show different screen shots of a possible software implementation of the method of the invention.

### Description of a preferred embodiment of the invention

The invention is related to a method and system for performing automatic allocation of a particle beam in a facility such as the one shown in figure 1 (possibly with a different number of treatment rooms). A special feature of the invention concerns the automatic termination of the allocation, under predefined circumstances. As seen in figure 1, a Treatment Control Room (TCR1 to TCR4) is present next to each Treatment Room (TR1 to TR4). In every TCR/TR a Beam User may be active who may request the beam for use and use it for treating a patient. The beam user may launch the beam request or other communications from an interface screen in the TCR or from a second screen in the TR. For the purposes of this invention, it is not important whether two screens are available (one in the TCR and one in the TR). It could be sufficient to have one screen in the TCR. What is important is that the beam user is provided with at least one interface screen from which to communicate with the Main Control Room computer. In the main control room, the Beam Operator is present and may supervise the automatic allocation. The Beam Operator is equally sitting before an interface screen, allowing him to communicate with the different Beam Users in the different TR/TCR's. According to the preferred embodiment, the facility can still be operated in manual mode, i.e. with manual allocation of the beam to the treatment rooms, as known in the art. The software of the invention allows flexible switching by the Beam Operator, between automatic mode and manual mode: The method of the invention is exclusively concerned however with the automatic mode.

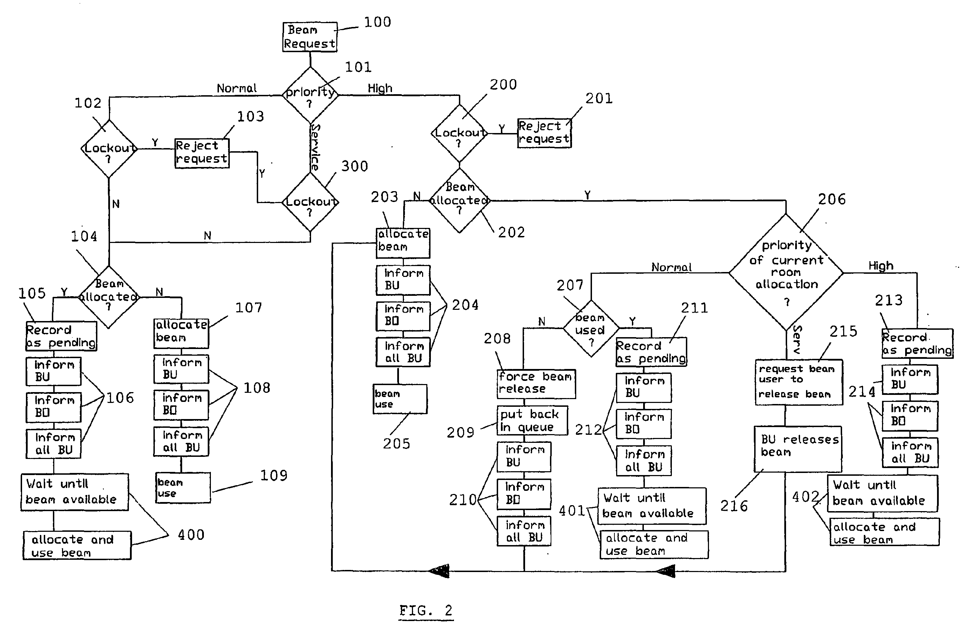

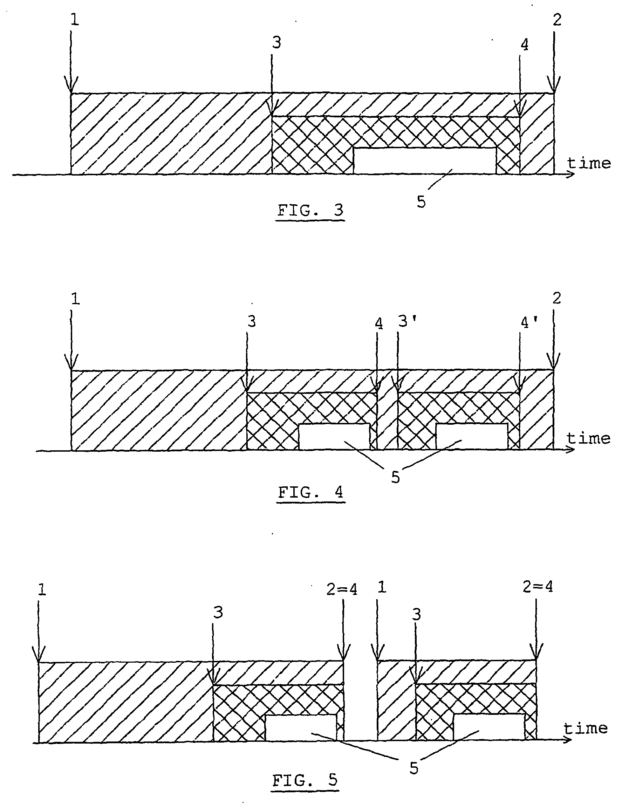

The operation of this automatic mode is illustrated in the flow diagram of figure 2.
A number of definitions are given first :
- the 'system' : this is defined as the totality of means enabling the allocation of the beam to a treatment room, and the treatment of a patient in said room. The system of the invention is characterized by the means to allocate the beam automatically.
- Room is 'allocated' : this refers to the period from the moment 1 on which the system selects the beam line until the beam release 2 (see fig. 3). During the allocation period, the beam can only be used in the room to which it is allocated. The beam release at point 4 is linked to the priority level of a particular beam request, as will be explained further in this description.
- Beam is 'used' : this refers to the period from the moment 3 on which the user asks a beam tuning until the end of irradiation 4 (see also fig. 3). Within a period of beam use, the actual field 5 takes place.
- 'Beam Operator (BO)' : the MCR operator responsible to supervise or to control the beam (allocation, delivery, ...).
- 'Beam User (BU)' : a Radiotherapist or anybody who uses the beam. The beam user is present in one of the treatment (control) rooms.

As seen in figure 2, the method of the invention starts with a beam request 100, coming from one of the Beam Users in one of the TR/TCRs. Each request is given a priority level by the person making the request. According to the preferred embodiment, three levels are defined : high, normal and service priority. The sowtware of the MCR computer checks (101) the level of priority of the incoming request.

A high priority is given to any urgent treatment for which the beam needs to be allocated to a particular room as quickly as possible. A high priority may for example be given to the treatment of a child under anaesthesia. A normal priority is given for any standard treatment which does not require immediate allocation, unless the beam is available anyway.

The service priority is the lowest level of priority and represents a special case. The beam can be requested by a beam user with a service priority when maintenance or other technical interventions need to be done in the TR. Before the service priority request can be launched, the beam user has to switch the TR to 'service mode' which defines a different set of parameters than the 'treatment mode' during which normal and high priority requests can be done. Therefore, service priority will be described in a somewhat separate way from the normal treatment sequences. It is also possible for the complete system to be put in the 'service mode', by the beam operator. In this system service mode, automatic allocation of the beam towards one of the rooms is disabled, so the method of the invention doesn't really apply to this mode. The special features related to these three priority levels will be described later in the description.

For a request with a Normal priority, a check 102 is made first, to find out whether the room issuing the request is not in Lockout mode. If it is, the beam request is rejected (103) by the system and a message is sent back to the beam user. Lockout mode is a mechanism used to prevent allocation of the beam to a (or several) treatment room(s). This lock is activated or deactivated in a configuration file, for each TR individually. A TR may for example be put in Lockout mode during maintenance or upgrade.

If the room is not in Lockout mode, the software of the MCR computer must check (104) whether or not the beam is already allocated to one of the other treatment rooms. If so, the request is recorded as pending (105), and the BU of the requesting room, the BO, and the other BU's are informed (106) of the new pending request, and the beam is allocated and used as soon as it becomes available (400). If not, the software of the MCR computer automatically allocates the beam (107) to the requesting room, whose BU is informed of this (108), as are the BO, and other BU's. At this point, the beam may be used (109).

When the beam request has a high priority, the system equally checks (200) whether the room is in lockout mode. If so, the request is rejected (201). If not, a check 202 is performed by the software of the MCR whether or not the beam is already allocated to another room.

If the beam is not already allocated, the automatic allocation takes place (203), and the BU's and BO are informed of this (204), after which the beam can be used (205). If the beam is already allocated, a further check (206) is made on the priority of the request for which the beam is allocated at that time. If this priority is normal, a further check (207) is made as to whether the beam is actually in use (figure 3, between points 3 and 4). If not, the release of the beam is automatically forced (208) by the software of the MCR computer, and the request for which the beam had been allocated is put back in the waiting list (209) (in a place defined by its priority level). BU's and BO are informed (210) of the beam release; and the beam is allocated (203) to the room issuing the high priority request. If the beam was in use, the high priority request is recorded as pending (211), and BU's and BO are informed of the pending request (212). In this case, the request will be put as high as possible in the waiting list of pending requests, preceding all other non-high priority requests, and will be processed as soon as the previous high-priority requests have been finished and the beam becomes available (401).

If the request for which the beam was already allocated had a high priority, the new request is automatically recorded as pending (213), and will take a place in the waiting list of pending requests preceding all pending non-high priority requests. All parties are informed of the pending request (214). The beam is allocated and used as soon as it becomes available (402).

If the request for which the beam was allocated had a service priority, the system requests a beam release (215) from the room in question The beam in service request can only be released by the beam user in the TCR since, in service request, there is no predefined process followed and it is not known when the beam can be released as this depends on whether the beam user will have finished his calibration or experimentation. The beam user in the TR/TCR will receive a message from the MCR, for example a pop-up window possibly accompanied by an audio signal, informing him of the MCR's request to release the beam. As soon as the beam user has released the beam (216), the beam is allocated (203) to the room from which the high-priority request was received.

When the beam request 100 has a service priority, once again, the lockout check is performed first (300), and the request is rejected (103) when the room is in Lockout mode. If this is not the case, the steps (104, 105, 107) of the procedure for normal priority will be followed.

The invention is related to a software tool able to manage the steps described above and in the flow diagram of figure 2.

Preferably, the software includes a means for switching between the automatic mode, which is basically the method of the invention, and the manual mode, which is the known method. In the manual mode, all steps which are performed automatically in the scheme of figure 2, such as beam allocation, and forced beam release, are commanded by the Beam Operator in the Main control room. It is of course desirable to have a system which can switch between the two modes, to allow intervention by the BO at all times. In automatic mode however, the system will work more efficiently, waiting times will be reduced, and the risk of error is minimized.

Whenever in the diagram of figure 2, a request is recorded as pending (105,211,213), the request is added to a waiting list of pending requests. At all times, the high priority requests are at the top of that list, followed by the normal priority requests, and the service priority requests. One could say that the system actually makes a separate list for each of the three priority levels. Every new request that must be put on hold will enter the appropriate list at the end. High priority requests are always accepted before any other normal or service priority request, but not before a previously pending high priority request. Normal priority requests are only accepted when the high priority list is empty, while service requests are accepted when both the high and normal priority waiting lists are empty.

The method shown in the flow diagram of figure 2 is related to the way in which an incoming request is handled. Once a request is put on the waiting list, this part of the method ends. However, the method is also related to the automatic allocation of a request which has spent a certain amount of time on the list. This automatic allocation takes place by the system of the invention, when there is no request with a higher priority level in said list, or no requests with the same priority level but received earlier, are preceding said request in said list. In other words, as soon as a request with a normal priority comes on top of the `normal priority' list, and no high priority is pending, the normal priority request is accepted automatically, as soon as the beam becomes available : the beam is then allocated automatically to the requesting room.

According to the preferred embodiment, after the beam has been allocated to a room, the actual beam use, between points 3 and 4 (figure 3) takes place according to a known method, involving beam tuning and the actual field duration 5.

After the treatment, the method of the invention is finished by the release of the beam. This beam release according to the invention, depends on the priority level of the original request.

Figure 3 shows a beam allocation with a service priority, wherein one field 5 is performed. At the end of the beam use period (point 4), the beam remains allocated to the room. The beam release 2 can only be initiated by the beam user.

Figure 4 shows a high priority request with two different field, wherein the configuration needs to be changed in between fields. The beam user ends the first beam use period at point 4, but because the priority is high, the system automatically continues to allocate the beam to this room. At point 3', the second treatment starts, ended at point 4', after which the beam is released at point 2 (end of beam allocation period). These procedures show that during high priority requests, it is primarily the beam user who decides when the beam use period as well as the beam allocation period are to be terminated. In the case of figure 4, the beam user might release the beam between beam use periods, if the change of the TR-configuration is expected to take a long time. In that case, the beam might be allocated to another room in between the two fields 5.

For a normal priority request, the system of the invention terminates the allocation period automatically, at the end of a beam use period, see figure 5. This allows the beam to become available automatically at the end of any normal priority treatment request. This feature helps to avoid unnecessary time delays.

During automatic beam scheduling mode, the beam operator is capable of changing the order of the pending requests of the same priority. However, it is not possible to put a normal priority request before a high priority request for example. Also, the BO can flush certain pending requests, i.e. remove them from the waiting list.

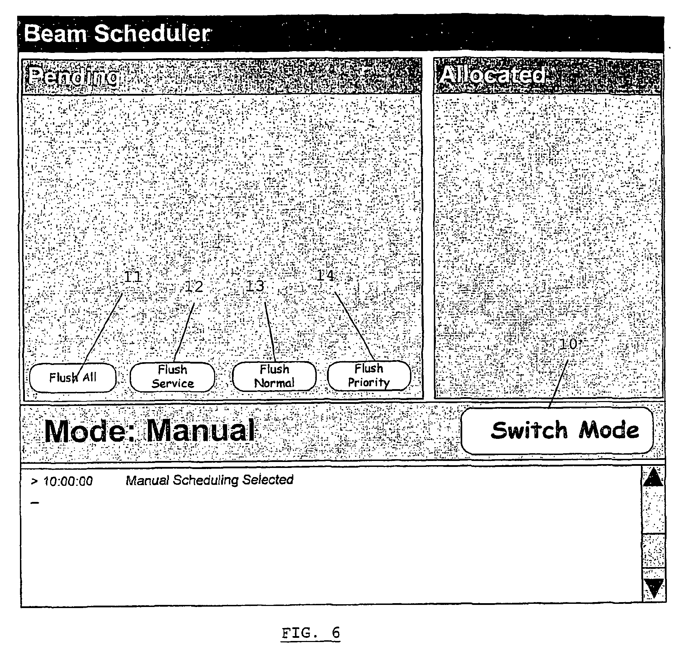

In the following pages, a number of possible interface screens for the Beam operator are described, with reference to figures 6 to 11.

Fig. 6 shows the situation where there is no allocated treatment room and no beam request. The mode is manual. Button 10 allows the BO to switch between manual and automatic mode. A number of flush buttons 11 to 14 are present. Button 11 allows the BO to flush all pending requests. Button 12, 13 and 14 allow the BO respectively to flush requests with service, normal or high priority. This 'flush' capability is preferably present both during manual and automatic mode.
The upper-part of the screen is split in two sub-windows :
- The right part displays information about the allocated room
- The left part displays information about pending beam requests. This is the waiting list (queue) wherein incoming requests are entered depending on their priority level.
The lower-part of the screen gives the historic of all events related to beam scheduling.

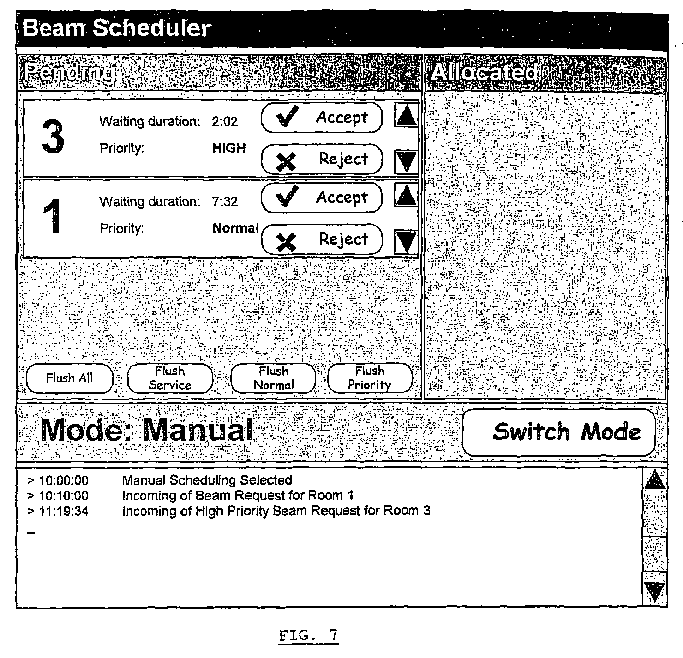

Fig. 7 shows the situation where treatment room 3 requests a high priority beam and room 1 a normal. The system is in Manual Beam Scheduling mode, and waiting for a Beam Operator request.
The system displays the information in the order of the automatic beam scheduling selection. So first the Highest priority requests, then the others with a First-In/First-Out policy.

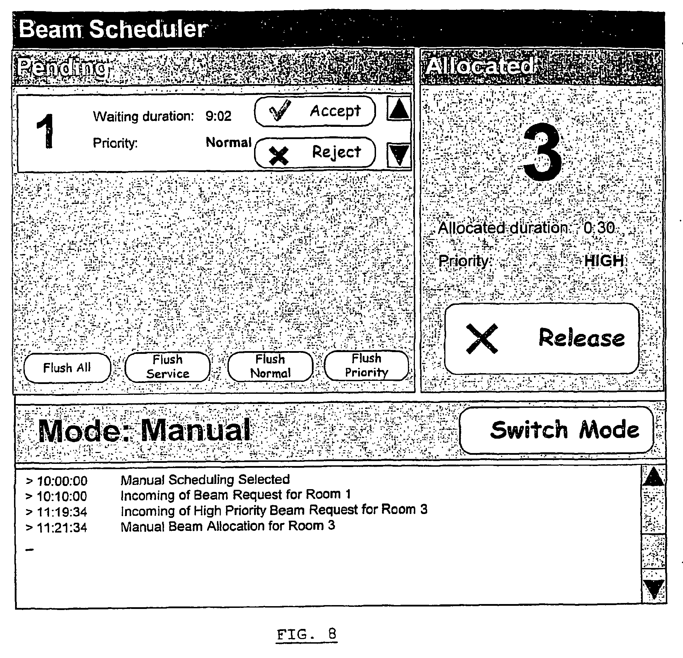

Fig. 8 shows the situation when the BO accepts the high priority request of treatment room 3. The request of room 1 is recorded as pending. Because there is an allocated beam, the Accept button of room 1 beam request is disabled.

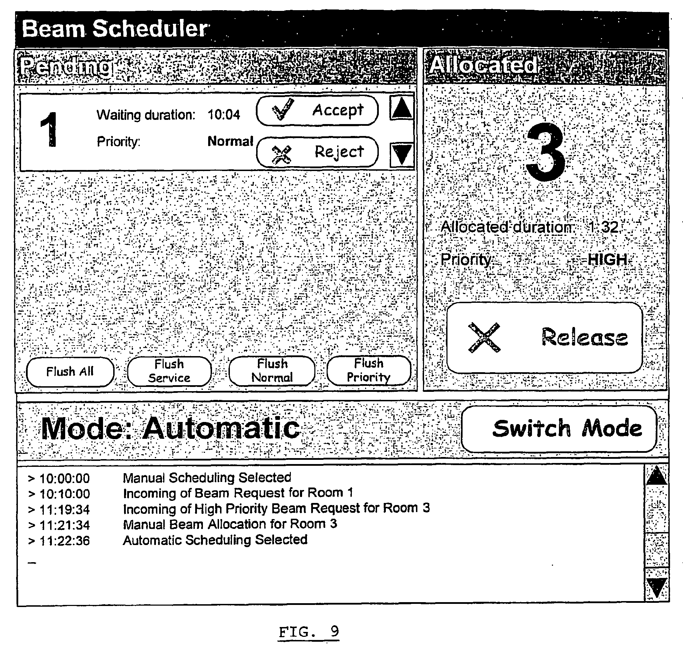

Fig. 9 shows the situation where the Beam Operator changes the Beam Scheduling Mode to Automatic. Nothing changes for allocated and pending requests, except that the Beam Operator can not Accept or Reject a Beam Request and can not Release the allocated beam. The 'Flush' buttons and the Step Down/Up in Order button (not shown) remain activated (similar as Manual Scheduling Mode).

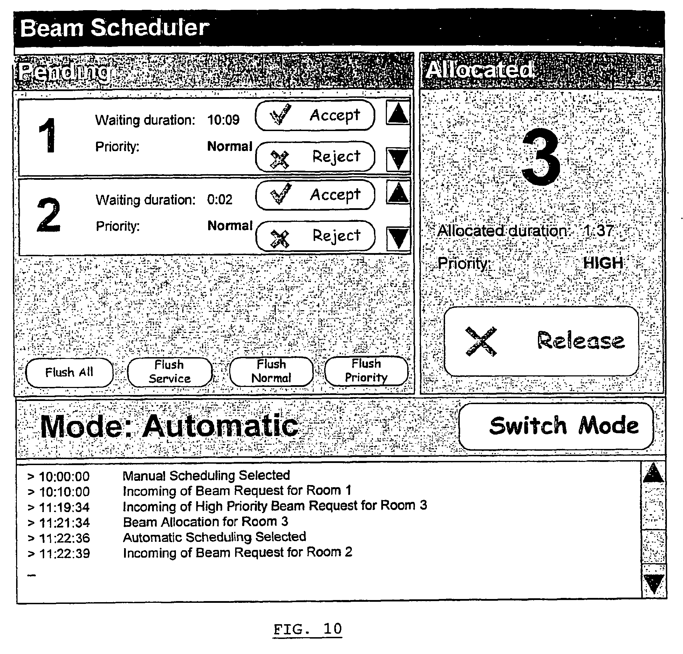

Fig. 10 shows the situation when room 2 requests the beam using normal priority. It is put in the waiting list of pending requests behind the already pending request from room 1.

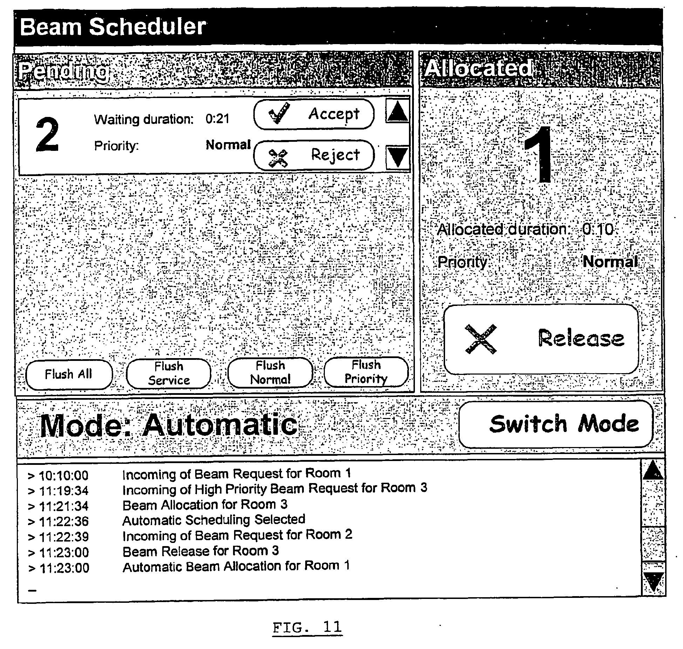

Fig. 11 shows the situation when room 3 releases the beam and room 1 is automatically allocated.

## Claims

1. A method for scheduling particle beam treatment operations in a facility comprising an irradiation source able to produce a beam, a plurality of treatment rooms (TR1 to TR4), a main control room (MCR) in connection with said treatment rooms, said facility being equipped with a system for allocating the beam to, using the beam in, and releasing the beam from one of said treatment rooms, **characterized in that** said method comprises the following steps :
- receiving a request from a treatment room, for allocation of the beam to said room, said request having a priority level,
- automatically performing the following steps :
- checking whether the beam is already allocated to a room,
- A : if the beam is not allocated then allocating the beam to the room from which said request was received,
- B : if the beam is allocated then either,
- B1 : forcing the release of the beam, and allocating the beam to the room from which said request was received, or
- B2 : putting said request on a waiting list, wherein said request is placed in a position on said list, said position being dependent on said priority level, and when no requests with a higher priority level are in said list, or no requests with the same priority level are preceding said request in said list, allocating the beam to the room from which said request was received, as soon as the beam becomes available, or
- B3 : requesting the beam user in said room to release the beam and, allocating the beam to the room from which said request was received, when said beam is released.

2. The method according to claim 1, wherein said beam is allocated during an allocation period and wherein, within said allocation period, said beam is used for irradiation during a use period, and wherein said method further comprises the step of automatically releasing the beam at the conclusion of said use period.

3. The method according to claim 2, wherein said priority level is chosen from the group of three priorities, given hereafter in growing order : service, normal, high, and wherein said step of automatically releasing the beam is only applied when said request has a normal priority.

4. The method according to claim 1, wherein case A or B2 applies and wherein
- said priority level is chosen from the group consisting of three priorities, given hereafter in growing order : service, normal, high, and wherein,
- the priority level of said request is checked and found to be normal or service.

5. The method according to claim 1, wherein case A applies and wherein
- said priority level is chosen from the group consisting of three priorities, given hereafter in growing order : service, normal, high, and wherein,
- the priority level of said request is checked and found to be high.

6. The method according to claim 1, wherein case B1 applies and wherein
- said priority level is chosen from the group consisting of three priorities, given hereafter in growing order : service, normal, high, and wherein,
- the priority level of said request is checked and found to be high.
- the priority of the already allocated beam is checked and found to be normal,
- the beam is checked whether in use and found not to be in use.

7. The method according to claim 1, wherein case B2 applies and wherein
- said priority level is chosen from the group consisting of three priorities, given hereafter in growing order : service, normal, high, and wherein,
- the priority level of said request is checked and found to be high.
- The priority level of the already allocated beam is checked and found to be normal,
- The beam is checked whether in use and found to be in use.

8. The method according to claim 1, wherein case B2 applies and wherein
- said priority level is chosen from the group consisting of three priorities, given hereafter in growing order : service, normal, high, and wherein,
- the priority level of said request is checked and found to be high.
- The priority level of the already allocated beam is checked and found to be high.

9. The method according to claim 1, wherein case B3 applies and wherein
- said priority level is chosen from the group consisting of three priorities, given hereafter in growing order : service, normal, high, and wherein,
- the priority level of said request is checked and found to be high,
- the priority level of the already allocated beam is checked and found to be service.

10. A computer program comprising program code means for performing the method of the preceding claims when said program runs on a computer.

11. **A computer program comprising program** code means stored on a computer readable medium for performing the method of the preceding claims, when said program runs on a computer.

12. A system for scheduling particle beam operations according to the method of any one of claims 1 to 9, comprising :
- a computer having a memory,
- at least one interface screen in every Treatment Control Room and/or every Treatment Room,
- at least one interface screen in the Main Control Room,
- a means for automatically checking the priority level of an incoming request,
- a means for automatically checking whether the beam is already allocated to a room,
- a means for automatically allocating the beam to a room,
- a means for automatically forcing a beam release from a room.
- a means for automatically requesting a beam user to release the beam.

## Patentansprüche

1. Verfahren für die Ablaufplanung von Teilchenstrahlenbehandlungsarbeitsvorgängen in einer Einrichtung, die Folgendes umfasst: eine Bestrahlungsquelle, die einen Strahl erzeugen kann, mehrere Behandlungsräume (TR1 bis TR4), einen Hauptkontrollraum (MCR = Main Control Room), der mit den Behandlungsräumen verbunden ist, wobei die Einrichtung mit einem System zur Zuordnung, Verwendung und Freisetzung des Strahls in einem der Behandlungsräume ausgestattet ist, **dadurch gekennzeichnet, dass** das Verfahren die folgenden Schritte umfasst:
- Empfangen einer Anforderung aus einem Behandlungsraum, den Strahl diesem Raum zuzuordnen, wobei diese Anforderung eine Prioritätsstufe besitzt,
- automatisches Durchführen der folgenden Schritte:
- Überprüfen, ob der Strahl bereits einem Raum zugeordnet wurde,
- A: wenn der Strahl noch nicht zugeordnet wurde, wird er dem Raum zugeordnet, aus dem die Anforderung kam,
- B: wenn der Strahl bereits zugeordnet wurde, wird entweder
- B1: die Freisetzung des Strahls erzwungen und der Strahl dem Raum zugeordnet, aus dem die Anforderung kam, oder
- B2: die Anforderung auf eine Warteliste gesetzt, wobei die Anforderung in der Liste eine von der Prioritätsstufe abhängende Position belegt, und wenn sich in der Liste keine Anforderungen mit höherer Prioritätsstufe befinden oder der Anforderung keine Anforderungen mit der gleichen Prioritätsstufe vorangehen, wird der Strahl, sobald er verfügbar ist, dem Raum zugeordnet, aus dem die Anforderung kam, oder
- B3: der Strahlbenutzer in dem Raum dazu aufgefordert, den Strahl freizusetzen, und der Strahl, wenn er freigesetzt worden ist, dem Raum zuzuordnen, aus dem die Anforderung kam.

2. Verfahren nach Anspruch 1, bei dem der Strahl im Verlauf eines Zuordnungszeitraums zugeordnet und innerhalb dieses Zuordnungszeitraumes im Verlauf eines Verwendungszeitraumes für die Bestrahlung verwendet wird, wobei das Verfahren weiter den Schritt umfasst, dass der Strahl bei Beendigung des Verwendungszeitraumes automatisch freigesetzt wird.

3. Verfahren nach Anspruch 2, bei dem die Prioritätsstufe aus einer Gruppe von drei Prioritäten ausgewählt wird, die nachfolgend in aufsteigender Reihenfolge angegeben sind: Instandhaltung, normal, hoch, und der zur automatische Strahlenfreisetzung Schritt nur angewendet wird, wenn die Anforderung eine normale Priorität aufweist.

4. Verfahren nach Anspruch 1, bei dem Fall A oder B2 zutrifft und
- die Prioritätsstufe aus einer Gruppe von drei Prioritäten ausgewählt wird, die nachfolgend in aufsteigender Reihenfolge angegeben sind: Instandhaltung, normal, hoch, und bei dem
- die Prioritätsstufe der Anforderung überprüft wird und entweder normal oder Instandhaltung entspricht.

5. Verfahren nach Anspruch 1, bei dem Fall A zutrifft und
- die Prioritätsstufe aus einer Gruppe von drei Prioritäten ausgewählt wird, die nachfolgend in aufsteigender Reihenfolge angegeben sind: Instandhaltung, normal, hoch, und bei dem
- die Prioritätsstufe der Anforderung überprüft wird und hoch entspricht.

6. Verfahren nach Anspruch 1, bei dem Fall B1 zutrifft und
- die Prioritätsstufe aus einer Gruppe von drei Prioritäten ausgewählt wird, die nachfolgend in aufsteigender Reihenfolge angegeben sind: Instandhaltung, normal, hoch, und bei dem
- die Prioritätsstufe der Anforderung überprüft wird und "hoch" entspricht,
- die Priorität des bereits zugeordneten Strahls überprüft wird und "normal" entspricht,
- der Teilchenstrahl daraufhin überprüft wird, ob er gerade verwendet wird, und festgestellt wird, dass er nicht verwendet wird.

7. Verfahren nach Anspruch 1, bei dem Fall B2 zutrifft und
- die Prioritätsstufe aus einer Gruppe von drei Prioritäten ausgewählt wird, die nachfolgend in aufsteigender Reihenfolge angegeben sind: Instandhaltung, normal, hoch, und bei dem
- die Prioritätsstufe der Anforderung überprüft wird und hoch entspricht,
- die Prioritätsstufe des bereits zugeordneten Strahls überprüft wird und normal entspricht,
- der Strahl daraufhin überprüft wird, ob er gerade verwendet wird, und festgestellt wird, dass er verwendet wird.

8. Verfahren nach Anspruch 1, bei dem Fall B2 zutrifft und
- die Prioritätsstufe aus einer Gruppe von drei Prioritäten ausgewählt wird, die nachfolgend in aufsteigender Reihenfolge angegeben sind: Instandhaltung, normal, hoch, und bei dem
- die Prioritätsstufe der Anforderung überprüft wird und hoch entspricht,
- die Prioritätsstufe des bereits zugeordneten Strahls überprüft wird und hoch entspricht.

9. Verfahren nach Anspruch 1, bei dem Fall B3 zutrifft und
- die Prioritätsstufe aus einer Gruppe von drei Prioritäten ausgewählt wird, die nachfolgend in aufsteigender Reihenfolge angegeben sind: Instandhaltung, normal, hoch, und bei dem
- die Prioritätsstufe der Anforderung überprüft wird und hoch entspricht,
- die Prioritätsstufe des bereits zugeordneten Strahls überprüft wird und Instandhaltung entspricht.

10. Computerprogramm, das ein Programmcodemittel umfasst, mit dem das Verfahren der vorangehenden Ansprüche ausgeführt werden kann, wenn das Programm auf einem Computer läuft.

11. Computerprogramm, das ein auf einem computerlesbaren Medium gespeichertes Programmcodemittel umfasst, mit dem das Verfahren der vorhergehenden Ansprüche ausgeführt werden kann, wenn das Programm auf einem Computer läuft.

12. System für die Ablaufplanung von Teilchenstrahlenbehandlungsarbeitsvorgängen gemäß dem Verfahren nach einem der Ansprüche 1 bis 9, das Folgendes umfasst:
- einen Computer mit einem Speicher,
- mindestens einen Schnittstellenbildschirm in jedem Behandlungskontrollraum und/ oder jedem Behandlungsraum,
- mindestens einen Schnittstellenbildschirm im Hauptkontrollraum,
- ein Mittel, mit dem die Prioritätsstufe einer eintreffenden Anforderung automatisch überprüft werden kann,
- ein Mittel, mit dem automatisch überprüft werden kann, ob der Strahl bereits einem Raum zugeordnet wurde,
- ein Mittel, mit dem der Strahl automatisch einem Raum zugeordnet werden kann,
- ein Mittel, mit dem automatisch die Freisetzung eines Strahls von einem Raum erzwungen werden kann,
- ein Mittel, mit dem ein Strahlbenutzer automatisch dazu aufgefordert werden kann, den Strahl freizusetzen.

## Revendications

1. Procédé pour planifier des opérations de traitement par faisceaux de particules dans une installation, comprenant une source de rayonnement capable de produire un faisceau, une pluralité de salles de traitement (TR1 à TR4), un salle de commande principale (main control room/MCR) reliée auxdites salles de traitement, ladite installation étant munie d'un système pour attribuer le faisceau à, utiliser le faisceau dans et libérer le faisceau de l'une desdites salles de traitement, **caractérisé en ce que** ledit procédé comprend les étapes suivantes :
- la réception d'une demande de salle de traitement, pour attribuer le faisceau à ladite salle, ladite demande ayant un certain niveau de priorité,
- la réalisation automatique des étapes suivantes :
- la vérification pour savoir si le faisceau est déjà attribué à une salle,
- A : si le faisceau n'est pas attribué, l'attribution du faisceau à la salle d'où a été reçue ladite demande,
- B : si le faisceau est attribué,
- B1 : la libération forcée du faisceau et l'attribution du faisceau à la salle d'où a été reçue la demande ; ou
- B2 : le dépôt de ladite demande sur une liste d'attente, sur laquelle ladite demande est placée dans une certaine position de ladite liste, ladite position dépendant dudit niveau de priorité et, lorsqu'aucune demande d'un niveau de priorité supérieur ne se trouve dans ladite liste ou qu'aucune demande de même niveau de priorité ne précède ladite demande dans ladite liste, l'attribution du faisceau à la salle d'où est reçue ladite demande, dès que le faisceau devient disponible ; ou
- B3 : la demande à l'opérateur de faisceaux dans ladite salle qu'il libère le faisceau et l'attribution du faisceau à la salle d'où est reçue ladite demande lorsque ledit faisceau est libéré.

2. Procédé selon la revendication 1, dans lequel ledit faisceau est attribué au cours d'une période d'attribution, dans lequel, durant ladite période d'attribution, ledit faisceau est utilisé pour une irradiation au cours d'une période d'utilisation et dans lequel ledit procédé comprend en outre l'étape de libération automatique du faisceau à la conclusion de ladite période d'utilisation.

3. Procédé selon la revendication 2, dans lequel ledit niveau de priorité est choisi dans le groupe de trois priorités, indiquées ci-après dans l'ordre croissant : maintenance, priorité normale, priorité élevée, et dans lequel ladite étape de libération automatique du faisceau n'est appliquée que lorsque ladite demande a une priorité normale.

4. Procédé selon la revendication 1, dans lequel le cas A ou B2 s'applique, dans lequel
- ledit niveau de priorité est choisi dans le groupe constitué de trois priorités, indiquées ci-après dans l'ordre croissant : maintenance, priorité normale, priorité élevée, et dans lequel
- le niveau de priorité de ladite demande est vérifié et est identifié comme normal ou maintenance.

5. Procédé selon la revendication 1, dans lequel le cas A s'applique, dans lequel
- ledit niveau de priorité est choisi dans le groupe constitué de trois priorités, indiquées ci-après dans l'ordre croissant : maintenance, priorité normale, priorité élevée, et dans lequel
- le niveau de priorité de ladite demande est vérifié et est identifié comme élevé.

6. Procédé selon la revendication 1, dans lequel le cas B1 s'applique, dans lequel
- ledit niveau de priorité est choisi dans le groupe constitué de trois priorités, indiquées ci-après dans l'ordre croissant : maintenance, priorité normale, priorité élevée, et dans lequel
- le niveau de priorité de ladite demande est vérifié et est identifié comme élevé,
- la priorité du faisceau déjà attribué est vérifiée et est identifiée comme normale, et
- le faisceau est vérifié pour contrôler s'il est utilisé et est identifié comme n'étant pas en cours d'utilisation.

7. Procédé selon la revendication 1, dans lequel le cas B2 s'applique et dans lequel
- ledit niveau de priorité est choisi dans le groupe constitué de trois priorités, indiquées ci-après dans l'ordre croissant : maintenance, priorité normale, priorité élevée, et dans lequel
- le niveau de priorité de ladite demande est vérifié et est identifié comme élevé,
- le niveau de priorité du faisceau déjà attribué est vérifié et est identifié comme normal, et
- le faisceau est vérifié pour contrôler s'il est utilisé et est identifiée comme étant en cours d'utilisation.

8. Procédé selon la revendication 1, dans lequel le cas B2 s'applique et dans lequel
- ledit niveau de priorité est choisi dans le groupe constitué de trois priorités, indiquées ci-après dans l'ordre croissant : maintenance, priorité normale, priorité élevée, et dans lequel
- le niveau de priorité de ladite demande est vérifié et est identifié comme être élevé, et
- le niveau de priorité du faisceau déjà attribué est vérifié et est identifié comme élevé.

9. Procédé selon la revendication 1, dans lequel le cas B3 s'applique et dans lequel
- ledit niveau de priorité est choisi dans le groupe constitué de trois priorités, indiquées ci-après dans l'ordre croissant : maintenance, priorité normale, priorité élevée, et dans lequel
- le niveau de priorité de ladite demande est vérifié et est identifié comme élevé, et
- le niveau de priorité du faisceau déjà attribué est vérifié et est identifiée comme étant maintenance.

10. Programme informatique comprenant des moyens de codage de programme afin de mettre en oeuvre le procédé des revendications précédentes lorsque ledit programme tourne sur un ordinateur.

11. Programme informatique comprenant des moyens de codage de programme stockés sur un support lisible par ordinateur afin de mettre en oeuvre le procédé des revendications précédentes, lorsque ledit programme tourne sur un ordinateur.

12. Système de programmation de l'utilisation de faisceaux de particules selon le procédé de l'une quelconque des revendications 1 à 9, comprenant :
- un ordinateur ayant une mémoire,
- au moins un écran d'interface dans chaque salle de commande de traitement et/ou dans chaque salle de traitement,
- au moins un écran d'interface dans la salle de commande principale,
- un moyen pour vérifier automatiquement le niveau de priorité d'une demande entrante,
- un moyen pour vérifier automatiquement si le faisceau est déjà attribué à une salle,
- un moyen pour attribuer automatiquement le faisceau à une salle,
- un moyen pour forcer automatiquement une libération de faisceau d'une salle, et
- un moyen pour demander automatiquement à un opérateur de faisceaux de libérer le faisceau.
